# EUROPEAN PATENT APPLICATION

(11) **EP 2 236 493 A1**
(43) Date of publication of application: **06.10.2010**
(21) Application number: 10151783.7
(22) Date of filing: 27.01.2010
(51) Int. Cl.: C07C 303/24, C07C 305/10

(54) **Preparation of alkoxysulfates**

(71) Applicant: Shell Internationale Research Maatschappij B.V., 2596 HR The Hague (NL)
(72) Inventor: Krijger, Martien, 1031 HW, Amsterdam (NL); Meurs, Jan Hermen Hendrik, 1031 HW, Amsterdam (NL); Smit, Jasper Roelf, 1031 HW, Amsterdam (NL); Van Zon, Arie, 1031 HW, Amsterdam (NL)

(57) **Abstract**

The present invention relates to the use of an alkylene sulfate for reducing the level of at least one undesirable by-product in the formation of alkoxysulfates from one or more nucleophile.

## Description

### Field of the Invention

The present invention relates to a process for the preparation of alkoxysulfates.

### Background of the Invention

A large variety of products useful, for instance, as surfactants and chemical intermediates are prepared by the alkoxylation and subsequent sulfation of compounds having one or more nucleophilic groups. These include alkoxysulfates according to general formula (I).

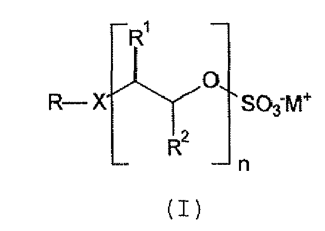

In the general formula (I) R refers to any suitable group or groups, and X refers to oxygen, nitrogen or sulfur, such that R-XH is a nucleophile. R¹ and R² are, independently, hydrogen or an alkyl group and n corresponds to the number of alkoxy groups present in the molecule. Such a formula may also be used to indicate a mixture of alkoxysulfates. In this case, n refers to the average number of alkoxy groups per molecule. M is a metal.

Related compounds, of general formula (II), based on tertiary amines, are also of interest (see G. Jakobi and A. Löhr, Detergents and Textile Washing, Principles and Practice, VCH Publishers, Weinheim, Germany, 1987). These compounds, of are of use as zwitterionics or general amphoteric surfactants in household detergents and in enhanced oil recovery (EOR). In general formula (II) R⁰-N refers to a tertiary amine. Such molecules are not simple to produce using standard chemical methods.

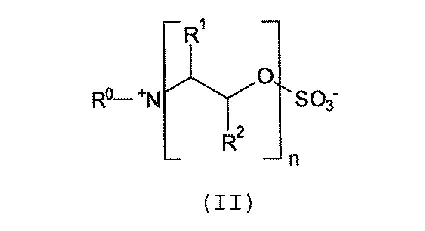

Generally such molecules, or mixtures of molecules, of general formula (I) are produced by reacting the parent nucleophile or mixture of nucleophiles (general formula III), with a number of equivalents of the relevant alkylene oxide(s), in the presence of a catalyst, in order to produce a mixture of alkoxylates (general formula IV) containing an average number of alkoxy groups, n. The resultant alkoxylates can then be sulfated to provide the desired alkoxysulfate product mixture (I). Again, this product will be a mixture of compounds containing an average number of alkoxy groups, n.

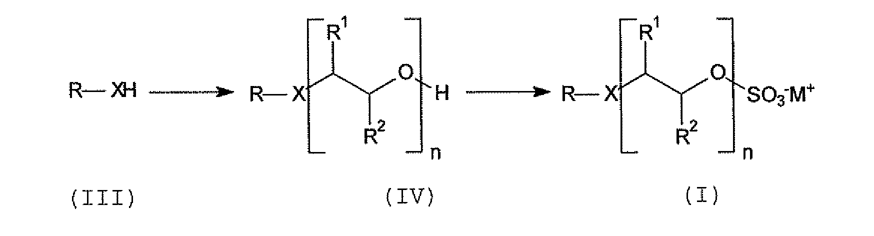

The range of alkoxylates formed in the above-described alkoxylation reactions will depend on the starting material nucleophile(s) and the conditions and catalysts used for the alkoxylation. Known catalysts for alkoxylation include basic metal hydroxide compounds, such as potassium hydroxide. Acidic catalysts, such as Lewis acid and Brønsted acid catalysts are also known as alkoxylation catalysts. Alkoxylation processes catalysed by phosphate salts of the rare earth elements have also been described in the art (e.g. US-A-5057627 and WO 02/047817). A further class of alkoxylation catalysts comprises the so-called double metal cyanide catalysts described in WO01/04183 and WO02/42356.

In any product distribution of a particular average, n, there will be some products that are more desirable than others for a specific application. For certain applications, it is, therefore, often desirable to provide a product distribution with as narrow a range of n values as possible.

Alkoxy groups are included in detergent molecules in order to improve detergent properties, in particular to increase the calcium tolerance of a specific detergent.

The number, n, of alkoxy groups present will affect the detergent properties and a specific product (i.e. with a specific value or average value of n) may, therefore, be tailored around the requirements of the product. In many cases, it is desirable to form compounds of general formula (I) in which n is low, preferably 3 or below, and most preferably 1. These latter, most preferred, compounds are usually made by reacting the parent nucleophile, such as an alcohol, with the relevant alkylene oxide in an approximately 1:1 ratio in the presence of a catalyst. However, all such known processes lead to the formation of a distribution of products containing high levels of non-alkoxylated products as well as a range of products in which n is greater than 1, the levels and ranges being dependent on the character of the starting nucleophile, the alkoxylation reaction conditions and the nature of the employed alkoxylation catalyst.

The problems involved in the alkoxylation of nucleophiles are even more pronounced when the starting nucleophile, or mixture of nucleophiles, comprises one or more secondary or tertiary alcohol(s), and particularly so when the alkylene oxide is ethylene oxide. The product of the ethoxylation of a secondary or tertiary alcohol is a primary alcohol. Such a product is inherently more reactive than the parent secondary or tertiary alcohol. Therefore, when alkoxylating such an alcohol with approximately one equivalent of ethylene oxide, the product mixtures of alkoxylates, and thus, after sulfation, the alkoxysulfates, will contain relatively high levels of compounds where n is greater than 1 and also a large amount of product where n is 0 and a relatively low amount of the desired (n=l) product.

Sulfation of alkoxylates is generally carried out by reaction with SO₃ in a falling film reactor, leading to the formation of alkoxysulfates (I). When sulfating the products of an alkoxylation reaction, the sulfation product will be a mixture of alkoxysulfates with a distribution of n values and will also contain sulfate(s), formed by sulfation of the residual (or unreacted) alcohol(s) present in the alkoxylation product mixture. The presence of the latter component(s) (i.e. formula (I), where n = 0, X = oxygen) in the product mixture can be detrimental in terms of product characteristics such as calcium tolerance and, hence, detergency performance and also handleability, due to the high Krafft point and high melting point of the alcohol sulfate.

The formation of 1,4-dioxanes (also known as p-dioxanes) during sulfation is another problem encountered in the production of alkoxysulfates, limiting the flexibility of this process. The presence of the noxious p-dioxane is non-desirable in detergents, particularly in personal care products, such as shampoos. By the use of a falling film sulfation reactor and a swift and thorough neutralization technique the residence times of the alkoxylate and alkoxysulfate in (Lewis) acidic medium can be kept to a minimum. Hereby the SO₃-catalyzed backbiting of the alkoxylate chain can be overcome, but only at an increased production cost.

Furthermore, the sulfation of alkoxylates of general formula (IV), wherein X is oxygen, particularly if the parent alcohol (III) is a secondary or a tertiary alcohol, and wherein R¹ is hydrogen or an alkyl group and R² is an alkyl group, by SO₃ is difficult as it may give rise to olefin and sulfuric acid formation due to the occurrence of an acid-catalyzed elimination reaction. Such products are also undesirable constituents of the final product mixture.

It would therefore be desirable to provide an alkoxysulfate composition of the general formula (I) or (II), wherein n is a low number, preferably 3 or below, and most preferably 1, and wherein said composition comprises a reduced amount of the by-products usually associated with the production of alkoxysulfates from nucleophiles, such as alcohols, by a two-step alkoxylation/sulfation process.

Furthermore, it would also be desirable to provide a facile method for the synthesis of compounds of general formula (II).

Alkylene sulfates are known in the art as synthetic reagents in the preparation of surfactant molecules.

WO 96/35663 describes the preparation of oligomeric alkylene sulfates using ethylene sulfate.

Gautun, O. R., et al. Acta Chemica Scandinavica, 1996, 50, 170-177 discloses the selective synthesis of aliphatic ethylene glycol sulfonates. This document describes the use of ethylene sulfate as a replacement for 'epoxide synthons' in the iterative addition of alkoxy groups in a surfactant molecule.

A similar use of ethylene sulfate is described in both Rist, ∅., et al. Molecules, 2005, 10, 1169 and Rist, ∅., et al. Synthetic Communications, 1999, 29(5), 749-754.

### Summary of the Invention

According to the present invention there is provided the use of an alkylene sulfate for reducing the level of at least one undesirable by-product in the formation of alkoxysulfates from one or more nucleophile.

### Detailed Description of the Invention

It has now surprisingly been found that alkylene sulfates can successfully be used in the preparation of alkoxysulfates from a nucleophile in order to reduce the amount of undesirable by-products formed in the preparation of alcohol alkoxysulfates from said nucleophile.

The term 'alkylene sulfate' as used herein refers to 5-membered ring compounds of the general formula (V) and 6-membered ring compounds of the general formula (VI). Preferably the alkylene sulfate is a 5-membered ring compound of the general formula (V).

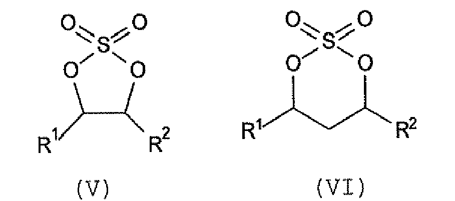

In general formulae (V) and (VI), R¹ and R² may be the same or different and are each, independently, selected from the group consisting of hydrogen and alkyl groups.

Typically, R¹ and R² are selected from the group consisting of hydrogen and short-chain alkyl groups. However, alkoxysulfates containing longer chain alkyl groups, such as those described in Rist, ∅., et al, Molecules, 2005, 10, 1169 may also be used.

As used herein, short-chain alkyl groups refers to alkyl groups of in the range of from 1 to 4 carbon atoms, preferably in the range of from 1 to 3 carbon atoms, more preferably in the range of from 1 to 2 carbon atoms, most preferably 1 carbon atom.

The choice of alkylene sulfate depends on the alkoxysulfate to be produced. Preferably, the alkylene sulfate is one in which R¹ is hydrogen and R² is hydrogen or a short chain alkyl group, more preferably R¹ is hydrogen and R² is hydrogen or a short chain alkyl group of in the range of from 1 to 2 carbon atoms. Most preferably, the alkylene sulfate is ethylene sulfate, i.e. general formula (V) wherein R¹ and R² are both hydrogen, or 1,2-propylene sulfate, i.e. general formula (V) wherein R¹ is hydrogen and R² is methyl, or its isomeric 6-membered ring compound, 1,3-propylene sulphate, i.e. general formula (VI) wherein R¹ and R² are hydrogen.

Such alkylene sulfates may be produced by any method known in the art. Suitable methods are described in FR 2664274 and B.B. Lohray, Synthesis, 1992, 1035-1105.

The alkoxysulfates of the present invention formed via reaction of alkylene sulfates of general formula (V) are of the general formula (I) or (II). Related products, containing a further -CH₂- group within the alkoxylate moiety may be formed when using alkylene sulfates of general formula (VI).

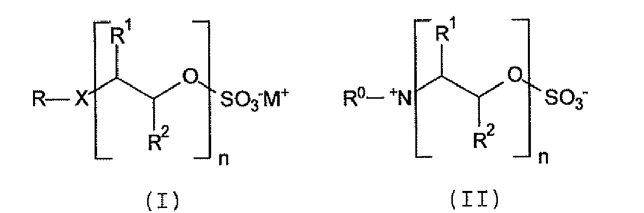

As stated above, in the general formula (I) R refers to any suitable group or groups, and X refers to oxygen, nitrogen or sulfur, such that R-XH is a nucleophile, and M is a metal. R⁰-N is a tertiary amine and thus R⁰ refers to three groups. These groups may be the same or different and may be any group as defined herein for R. R¹ and R² are as defined above. Preferably, R refers to a group having a linear or branched hydrocarbyl backbone. Such a hydrocarbyl backbone, or the branches thereof, may contain non-hydrocarbyl substituents, such as groups containing oxygen, nitrogen or sulfur atoms.

The nucleophiles of general formula (III) (R-XH) suitably utilized in the process of the present invention include (but are not necessarily limited to) alcohols, phenols, thiols (mercaptans), amines, polyols, carboxylic acids, carboxylic acid amides, and mixtures thereof. Generally, X represents either an oxygen, sulfur or (substituted, e.g. amino) nitrogen atom.

Among the suitable carboxylic acids, particular mention may be made of the mono- and dicarboxylic acids, both aliphatic (saturated and unsaturated) and aromatic, and their carboxylic acid amide derivatives. Specific examples include lauric acid, myristic acid, palmitic acid, stearic acid, oleic acid, rosin acids, tall oil acids and terephthalic acid, and their carboxylic acid amide derivatives.

Among the suitable amines, particular mention may be made of primary, secondary and tertiary alkylamines and of alkylamines containing both amino and hydroxyl groups, e.g. N'N-di(n-butyl)-ethanol amine and tripropanolamine.

Among the suitable thiols, particular mention may be made of primary, secondary and tertiary alkane thiols having from 9 to 30 carbon atoms, particularly those having from 9 to 20 carbon atoms. Specific examples of suitable tertiary thiols are those having a highly branched carbon chain which are derived via hydrosulfurisation of the products of the oligomerisation of lower olefins, particularly the dimers, trimers, tetramers and pentamers of propylene and the butylenes. Secondary thiols are exemplified by the products of the hydrosulfurisation of the substantially linear oligomers of ethylene as are produced by the Shell Higher Olefins Process (SHOP process). Representative, but by no means limiting, examples of thiols derived from ethylene oligomers include the linear carbon chain products, such as 2-decanethiol, 3-decanethiol, 4-decanethiol, 5-decanethiol, 3-dodecanethiol, 4-decanethiol, 5-decanethiol, 3-dodecanethiol, 5-dodecanethiol, 2-hexadecanethiol, 5-hexadecanethiol, and 8-octadencanethiol, and the branched carbon chain products, such as 2-methyl-4-tridecanethiol. Primary thiols are typically prepared from terminal olefins by hydrosulfurisation under free-radical conditions and include, for example, 1-dodecanethiol, 1-tetradecanethiol and 2-methyl-1-tridecanethiol.

Among the phenols, particular mention may be made of phenol and alkyl-substituted phenols wherein each alkyl substituent has from 3 to 30 (preferably from 3 to 20) carbon atoms, for example, p-hexylphenol, nonylphenol, p-decylphenol, nonylphenol and didecyl phenol.

In a preferred embodiment, the nucleophile is a hydroxyl-containing reactant.

In another preferred embodiment, the nucleophile is selected from alcohols and carboxylic acid amides, and mixtures thereof.

The most preferred nucleophiles herein are alcohols. Suitable starting alcohols for use in the preparation of an alkoxylated alcohol composition herein include those known in the art for reaction with alkylene oxides and conversion to alkoxylated alcohol products, including both mono- and poly-hydroxy alcohols.

Acyclic aliphatic mono-hydric alcohols (alkanols) form a most preferred class of reactants, particularly the primary alkanols, although secondary and tertiary alkanols are also very suitably utilized in the process of the present invention. It is particularly useful to be able directly to form alkoxysulfates of secondary alcohols since secondary alcohols can be derived from relatively cheap feedstocks such as paraffins (by oxidation) or from short chain C₆-C₁₀ primary alcohols (by propoxylation). Suitable paraffins for producing secondary alcohols are, for example, those produced from Fischer-Tropsch technologies.

Preference can also be expressed for alcohols (R-OH) having from 9 to 30 carbon atoms, with C₉ to C₂₄ alcohols considered more preferred and C₉ to C₂₀ alcohols considered most preferred, including mixtures thereof, such as a mixture of C₉ and C₂₀ alcohols. As a general rule, the alcohols may be of branched or straight chain structure depending on the intended use. In one embodiment, preference further exists for alcohol reactants in which greater than 50 percent, more preferably greater than 60 percent and most preferably greater than 70 percent of the molecules are of linear (straight chain) carbon structure. In another embodiment, preference further exists for alcohol reactants in which greater than 50 percent, more preferably greater than 60 percent and most preferably greater than 70 percent of the molecules are of branched carbon structure.

Commercially available mixtures of primary monohydric alcohols prepared via the oligomerisation of ethylene and the hydroformylation or oxidation and hydrolysis of the resulting higher olefins are particularly preferred. Examples of commercially available alcohol mixtures include the NEODOL (NEODOL, as used throughout this text, is a trademark) alcohols, sold by Shell Chemical Company, including mixtures of C₉, C₁₀ and C₁₁ alcohols (NEODOL 91 alcohol), mixtures of C₁₂ and C₁₃ alcohols (NEODOL 23 alcohol), mixtures of C₁₂, C₁₃, C₁₄ and C₁₅ alcohols (NEODOL 25 alcohol), and mixtures of C₁₄ and C₁₅ alcohols (NEODOL 45 alcohol, and NEODOL 45E alcohol); the ALFOL (ALFOL is a trademark) alcohols (ex. Vista Chemical Company), including mixtures of C₁₀ and C₁₂ alcohols (ALFOL 1012), mixtures of C₁₂ and C₁₄ alcohols (ALFOL 1214), mixtures of C₁₆ and C₁₈ alcohols (ALFOL 1618), and mixtures of C₁₆, C₁₈ and C₂₀ alcohols (ALFOL 1620), the EPAL (EPAL is a trademark) alcohols (Ethyl Chemical Company), including mixtures of C₁₀ and C₁₂ alcohols (EPAL 1012), mixtures of C₁₂ and C₁₄ alcohols (EPAL 1214), and mixtures of C₁₄, C₁₆ and C₁₈ alcohols (EPAL 1418), and the TERGITOL-L (Tergitol is a trademark) alcohols (Union Carbide), including mixtures of C₁₂, C₁₃, C₁₄ and C₁₅ alcohols (TERGITOL-L 125). Also suitable for use herein is NEODOL 1, which is primarily a C₁₁ alcohol. Also very suitable are the commercially available alcohols prepared by the reduction of naturally occurring fatty esters, for example, the CO and TA products of Proctor and Gamble Company and the TA alcohols of Ashland Oil Company.

As mentioned above, secondary alcohols are also a preferred class of reactants for use herein. Examples of secondary alcohols suitable for use herein include 2-undecanol, 2-hexanol, 3-hexanol, 2-heptanol, 3-heptanol, 2-octanol, 3-octanol, 2-nonanol, 2-decanol, 4-decanol, 2-dodecanol, 4-tridecanol, 2-tetradecanol, 2-hexadecanol, 2-octadecanol and mixtures thereof.

Mixtures of alcohols comprising primary and secondary alcohols are also suitable for use herein.

In particular, oxidation products arising from Fischer-Tropsch derived paraffins (which may include mixtures of primary and secondary alcohols), as described in co-pending application US 60/983269, are particularly suitable for use herein.

In one embodiment of the present invention R-XH may be an alcohol (i.e. X = oxygen) and comprise one or more polyether chain(s), such as present in polyalkylene glycols, e.g. glycerol-based polypropylene glycols. The present invention is particularly useful for the preparation of compounds with one or more mono-ethoxylated polypropylene glycol chain(s), which can be formed by the hydrolysis of the alcohol ethoxysulfate(s) formed by reaction of the compounds having one or more polypropylene glycol chain(s) with ethylene sulfate.

In a particularly preferred embodiment of the present invention, R comprises a linear or branched hydrocarbyl backbone containing no non-hydrocarbyl substituents.

Regardless of the composition of R, R-XH may be a primary, secondary or tertiary nucleophilic organic compound. It is of particular advantage to apply the present invention when R-XH is a secondary or tertiary, preferably secondary, alcohol.

As stated above, when the nucleophile is a tertiary amine of the formula R⁰-N, R⁰ refers to any three groups as defined herein for R, with the proviso that R⁰-N is a tertiary amine. Tertiary amines of particular interest include tri-alkyl amines, where each alkyl group may be the same or different. Preferably, each alkyl group contains in the range of from 1 to 20 carbon atoms. In one particularly preferred group of tri-alkyl amines the nitrogen atom is substituted with two methyl groups and one alkyl group containing 12 to 18 carbon atoms.

As used herein, M refers to a metal. Preferably the metal is an alkali metal, even more preferably the metal is selected from sodium, lithium and potassium, most preferably the metal is sodium.

In another embodiment of the present invention, R-XH may be an alcohol which already contains alkoxy groups, i.e. of general formula (VII).

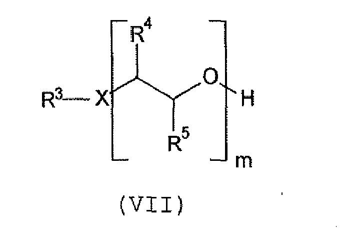

Herein, R³ refers to any suitable group or groups, and X refers to oxygen, nitrogen or sulfur, such that R³-XH is a nucleophile. Preferably, R³ refers to a group having a linear or branched hydrocarbyl backbone. Such a hydrocarbyl backbone, or the branches thereof, may contain non-hydrocarbyl substituents, such as groups containing oxygen, nitrogen or sulfur atoms. In a particularly preferred embodiment, R³ comprises a linear or branched hydrocarbyl backbone containing no non-hydrocarbyl substituents.

The skilled person will readily understand that in this embodiment, R³-XH may be any nucleophile as defined above for R-XH, with the exception, of course, of the embodiment wherein R³-XH is of the general formula (VII).

R⁴ and R⁵ are each, independently, hydrogen or an alkyl group, preferably hydrogen or a short-chain alkyl group. As used herein, short-chain alkyl groups refers to alkyl groups of between 1 and 4 carbon atoms, preferably between 1 and 3 carbon atoms, more preferably between 1 and 2 carbon atoms, most preferably 1 carbon atom. Preferably, R⁴ is hydrogen and R⁵ is selected from the group consisting of hydrogen and a short-chain alkyl group having between 1 and 2 carbon atoms (i.e. an methyl or ethyl group).

As used herein, the number m corresponds to the number of alkoxy groups present per molecule and is preferably in the range of from 1 and 70, more preferably in the range of from 1 and 50, even more preferably in the range of from 1 to 20. Alternatively, general formula (VII) may be used to indicate a mixture of compounds. In this case, m refers to the average number of alkoxy groups per molecule and may be any number greater than zero and no more than 70, preferably no more than 50, even more preferably no more than 20.

In each case, the number of alkoxy groups (m) may refer to a single type of alkoxy group or a mixture of two or more alkoxy groups. Such a mixture of alkoxy groups may include both random and block co-polymers of the alkoxy groups.

As used herein the term "reducing the level of at least one undesirable by-product" means reducing the level of one or more of the by-products usually associated with a two-step alkoxylation/sulfation process for the formation of alkoxysulfates from the corresponding nucleophiles. Such by-products include the residual nucleophile(s) (also called unconverted or free nucleophile(s)) and its sulphate(s), a distribution of heavily alkoxylated nucleophile(s) having more than 3 alkoxylate units per mole of nucleophile and their sulfates, and/or 1,4-dioxanes. If the nucleophile is an alcohol, such by-products include the residual alcohol(s) (also called unconverted or free alcohol(s)), the alcohol sulfate(s) (i.e. compounds of general formula (I) or (II), wherein n = 0 and X = oxygen), olefins derived from secondary alcohol(s) (upon sulfuric acid elimination from secondary alcohol sulfate(s)), a distribution of heavily alkoxylated alcohol(s) and their sulphate(s), and/or 1,4-dioxanes.

As used herein, reducing the amount of residual nucleophile, such as an alcohol, in the alkoxysulfate composition preferably means that the amount of residual nucleophile, such as an alcohol, in the alkoxysulfate composition prepared herein is no more than 40%, preferably no more than 30%, more preferably no more than 20%, even more preferably no more than 10% by weight of the alkoxysulfate composition.

As used herein, if the nucleophile is an alcohol, reducing the amount of alcohol sulfate in the alkoxysulfate composition preferably means that the amount of residual (free) alcohol sulfate in the alkoxysulfate composition prepared herein is no more than 20%, preferably no more than 10%, more preferably no more than 5%, even more preferably no more than 2% by weight of the alkoxysulfate composition.

As used herein, if the nucleophile is a secondary alcohol, reducing the amount of olefins derived from secondary alcohol sulfate(s) in the secondary alcohol alkoxysulfate composition preferably means that the amount of olefin in the secondary alcohol alkoxysulfate composition prepared herein is no more than 10%, preferably no more than 5%, more preferably no more than 2%, even more preferably no more than 1% by weight of the alkoxysulfate composition.

As used herein, reducing the level of 1,4-dioxanes means that the level of 1,4-dioxanes, i.e. depending on the nature of the alkoxylate chain 2,3,5,6-tetraalkyl-1,4-dioxane, 2,5-dialkyl-1,4-dioxane or 1,4-dioxane, present in the alkoxysulfate composition prepared herein is no more than 100 ppm, preferably no more than 10 ppm, more preferably no more than 5 ppm by weight of the alkoxysulfate composition.

The process for using an alkylene sulfate for the preparation of alkoxysulfates from the corresponding nucleophiles may be any suitable method known in the art. Methods known in the art include those described in Rist, ∅. et al., Molecules, 2005, 10, 1169; Rist, ∅. et al., Synthetic Communications, 1999, 29, 5, 749; and Carlson, P.H.J. et al, Acta Chem. Scand., 1996, 50, 170. Preference is given for methods in which the yield of alkoxysulfate is at least 50%, preferably at least 70%.

One particularly preferred process for the formation of alkoxysulfates, according to the present invention, is a two-phase process in which the nucleophile is dissolved in a water-miscible solvent and contacted with a base. The required alkylene sulfate is added, dissolved in a non-water-miscible solvent.

In this embodiment, suitable water-miscible solvents include, but are not limited to, those in the group consisting of tetrahydrofuran (THF), dimethylformamide (DMF), dimethylacetamide (DMA), hexamethylphosphoric triamide (HMPT) and sulphur-containing solvents. Preferably, the water-miscible solvent is selected from the group consisting of sulphur-containing solvents. More preferably, the water-miscible solvent is selected from the group consisting of sulphur-containing solvents, which comprise a sulfoxide or sulfone group. Even more preferably, the water-miscible solvent is selected from the group consisting of DMSO and sulfolane. Most preferably, the water-miscible solvent is DMSO.

Suitable non-water-miscible solvents include, but are not limited to, those in the group consisting of chlorinated solvents such as methylene chloride, chloroform, carbon tetrachloride, trichloroethane or chlorinated aromatics, such as chlorobenzene or dichlorobenzene, and non-chlorinated aromatics, such as toluene or xylenes. Preferably, the non-water-miscible solvent is selected from the group consisting of chlorinated hydrocarbons, such as methylene chloride, chloroform and trichloroethane. More preferably the non-water-miscible solvent is selected from the group consisting of methylene chloride and chloroform. Most preferably, the non-water-miscible solvent is methylene chloride.

In this embodiment, the base may be selected from alkali metals, alkali metal hydrides and hydroxides, carbonates or hydrogencarbonates of alkali metals or alkaline earth metals. It is particularly convenient if the base is selected from alkali metal hydroxides, which are cheap, easy to handle and readily available. Preferably, the base is selected from sodium, lithium or potassium hydroxide; most preferably the base is sodium hydroxide.

The reaction may be carried out at any suitable temperature or pressure. Preferably, the reaction is carried out at a temperature of at least -10 °C, more preferably at least 0 °C, even more preferably at least 10 °C. Preferably, the reaction is carried out at a temperature of at most 100 °C, more preferably at most 70 °C, even more preferably at most 40 °C.

Preferably, the reaction is carried out at a pressure of at least 10 kPa, more preferably at least 25 kPa, even more preferably at least 50 kPa. Preferably, the reaction is carried out at a pressure of at most 500 kPa, more preferably at most 250 kPa, even more preferably at most 150 kPa.

In a most preferred embodiment the reaction is carried out at ambient temperature and atmospheric pressure.

Reaction of a nucleophile with an alkylene sulfate, according to the present invention, will predominantly form the alkoxysulfate according to general formula (I) or (II), wherein n = 1. Thus, when applying the present invention, the formation of a distribution of molecules is avoided, and a single step procedure is used to replace a two-step procedure comprising two problematic reaction steps.

Following formation of the alkoxysulfate(s) according to the present invention, further chemical transformations may be carried out. For example, the alkoxysulfate(s) may be converted to alkoxysulfonates by reaction with sodium sulfite, or the alkoxysulfate(s) may be subjected to hydrolysis under acidic or neutral conditions to form alkoxylate(s).

The present invention will now be illustrated by the following non-limiting examples.

### Examples

NEODOL 45, a C14/C15 primary alcohol composition, is commercially available from The Shell Chemical Company. NEODOL 67, a C16/C17 primary alcohol composition is commercially available from The Shell Chemical Company. Heavy Detergent Feedstock (HDF) is a C₁₄-C₁₈ paraffin (GC analysis gives typically 25 wt% tetradecane, 24 wt% pentadecane, 23 wt% hexadecane, 21 wt% heptadecane and 6 wt% octadecane, of which approximately 7 wt% are predominantly methyl-branched C₁₄-C₁₉ paraffins; GC x GC analysis gives 240 mg/kg total mono-naphthenes, 0 mg/kg total di-naphthenes and 10 mg/kg total mono-aromatics).

The use of sodium hydride in a variety of inert solvents such as dimethylformamide (DMF), tetrahydrofuran (THF), acetonitrile, p-dioxane has been reported to give generally satisfactory conversions for ethoxysulfation of alcohols (also known as ethylsulfation), although low conversions have also been observed and reported for one type of primary alcohol, see Rist, 0., et al., Molecules, 2005, 10, 1169. However, we surprisingly found that the (rather expensive) reagent sodium hydride gave generally low-moderate yields (10-40%) in the ethylsulfation of primary and secondary alcohols such as NEODOL 45, NEODOL 67, and the mixture of secondary alcohols derived from C₁₄-C₁₈ paraffin, also known as Heavy Detergent Feedstock (HDF).

### Comparative Examples 1 to 7 and Examples 8 to 12

NEODOL 23, a C12/C13 primary alcohol composition, is commercially available from The Shell Chemical Company. Hexadecanol and 2-undecanol are available from Aldrich and 4-tridecanol is available from Chemical Samples Co, Columbus, Ohio, USA.

Comparative Examples 1 to 7 and Examples 8 to 12 of the present invention are carried out according to the following process unless otherwise indicated in Table 1. Under a nitrogen atmosphere the alcohol, solvent-1 and the base were reacted for 1 h under the conditions detailed in Table 1, in order to form a sodium alkoxylate mixture. Ethylene sulfate (available from Eastar Chemical Corporation, Sacramento, Ca, USA) dissolved in solvent-2 was added to the stirred sodium alkoxylate mixture at such a rate that the designated temperature could be maintained. The reaction mixture was then stirred at the indicated temperature overnight. At the times indicated in Table 1, small samples were taken. These samples were hydrolysed by treatment with 6N H₂SO₄ at 90°C for less than 1h and subsequently analysed by gas chromatography (GC).

GC was carried out on a Hewlett-Packard HP6890 apparatus with the following column: Varian-Chrompack capillary column CP-SM 5CB (low-bleed), length 50 m, internal diameter 0.25 mm, film thickness 0.4 µm and with the following temperature program: 125 °C (5 min); 125 - 325 °C (10 °C/min); 325 °C (5 min). Flame ionization detection and an internal normalization method of quantification were employed.

### Example 13: Ethoxysulfation of NEODOL 67 According to the Present Intention

NEODOL 67 (74.8 g, 300 mmol) and dichloromethane (60 ml) were added to a 3-necked round-bottomed flask (2-liter) equipped with a mechanical stirrer, a nitrogen inlet tube, a thermocouple and a dropping funnel (500-ml). Under a nitrogen atmosphere, a 50% suspension of sodium hydroxide (1.5 mol, 5.0 equivalents with respect to NEODOL 67) in dimethyl sulfoxide (DMSO) was added and the mixture was stirred for 15 minutes. The mixture was cooled to 15 °C and a solution of ethylene sulfate (48.4 g, 390 mmol, 1.3 eq with respect to NEODOL 67) in dichloromethane (400 ml) was added drop-wise at such a rate the reaction temperature did not exceed 25 °C (-1.5 ml/min). After complete addition the mixture was stirred at room temperature for an additional 2 hours. The conversion was 69% +/- 5%, due to broad overlapping peaks in the GC method, described in Examples 1-12 (Table 1).

To the reaction mixture was added demineralised water until phase separation occurred (-80 ml). The phases where separated and the upper phase was discarded. To the viscous lower layer was added demineralised water (250 ml). Two phases were formed. The clear and mobile lower layer was separated and extracted with demineralised water (2x 250 ml). The three combined viscous aqueous phases were filtered over a glass filter (porosity 4), then saturated with sodium chloride and subsequently extracted with isopropyl alcohol (5x 250 ml). The combined isopropyl alcohol layers were concentrated on a rotary evaporator and the residue was dried by azeotropic distillation with toluene (2x 250 ml) to yield 88.1 g of a white solid, sodium NEODOL 67 ethylsulfate (∼90% purity; the remainder being NEODOL 67). The yield of isolated sodium NEODOL 67 ethylsulfate was 200 mmol (67%).

**Table 1: Preparation of Alcohol Ethoxysulfates**

| Example | Alcohol (mmol) | Base (eq) | Solvent-1 | Ethylene sulfate^{a} (eq) | Solvent-2 | Temp (°C) | Time (h) | Conversion^{b} (%) | Remarks |
|---|---|---|---|---|---|---|---|---|---|
| 1* | Neodol 23 | KOH (0.05) | toluene | 1.0 | toluene | 90 | <½ | 12^{c} | prepared according to WO 96/035663 |
| | (50) | Na₂CO₃ (1.0) | water | | toluene | 40 | ½ | | |
| 2* | hexadecanol (20) | NaOH (1.0) | toluene | 1.0 | toluene | 20 | 24 | 10 | water removal with Dean-Stark setup at 130°C |
| 3* | Neodol 23 | Na (1.0) | p-dioxane | 1.0 | p-dioxane | 20 | ½ | 59 | deprotonation at |
| | (50) | | | | | 20 | 24 | 63 | 120°C for 20h |
| 4* | 4-tridecanol (50) | Na (1.15) | p-dioxane | 1.0 | p-dioxane | 20 | 2 | 54 | deprotonation at 120°C for 18h |
| 5* | Neodol 23 (50) | NaHCO₃ (1.0) | water | 1.0 | p-dioxane | 90 | 16 | 22^{d} | |
| 6* | 4-tridecanol | NaOH (1.5) | p-dioxane | 1.3 | CH₂Cl₂ | <25 | ½ | 0 | |
| | (50) | | | | | | 24 | 0 | |
| 7* | 4-tridecanol (50) | NaOH (1.5) | p-dioxane | 1.3 | p-dioxane | <25 | ½ | 0 | |
| 8 | Neodol 23 | NaOH (5.0) | DMSO | 1.0 | CH₂Cl₂ | 20-40 | ½ | 65 | |
| | (50) | | | | | 40 | 20 | 66 | |
| | | | | +0.5 | | 40 | 6 | 76 | |
| | | | | +0.5 | | <25 | ½ | 84 | |
| 9 | Neodol 23 | NaOH (1.5) | sulfolane | 1.0 | CH₂Cl₂ | <25 | ½ | 49 | minimum amount |
| | (50) | | | | | | 20 | 49 | CH₂Cl₂ to lower |
| | | | | | | | | | the viscosity. |
| 10 | 2-undecanol (50) | NaOH (1.5) | DMSO | 1.3 | CH₂Cl₂ | <25 | ½ | 48 | |
| 11 | 2-undecanol (50) | NaOH (5.0) | DMSO | 1.3 | CH₂Cl₂ | <25 | ½ | 57 | |
| 12 | Neodol 23 (50) | NaOH (5.0) | DMSO | 1.3 | CH₂Cl₂ | <0 0-20 | 24 | 69 | <0°C during addition; then slowly heated to 20°C |
| 13 | Neodol 67 (300) | NaOH (5.0) | DMSO | 1.3 | CH₂Cl₂ | <25 | 1 | -69 | |

| | | | | | | | | | |
|---|---|---|---|---|---|---|---|---|---|
| * Comparative Example. a. Ethylene sulfate is available from Eastar Chemical Corporation, Sacramento, Ca, USA and has been used without purification. b. Measured by GC after hydrolysis in 6N sulphuric acid at 90°C for <1h (wt% of 1EO-adduct on alcohol intake). c. Trace (<1%) of 2EO derivative also present. d. 2EO and 3EO derivatives also observed in GC after hydrolysis. | | | | | | | | | |

### Comparative Example 14

An ethoxylate derived from a C₁₂/C₁₃ primary alcohol composition and having an average of 1 EO group per molecule, prepared using potassium hydroxide as the ethoxylation catalyst, was analysed for average number of moles of EO per molecule and the amount of residual or unconverted (free) alcohols.

The analysis was carried out using gas chromatography (method UK.3743-94). In this method, the samples are first silylated using BSTFA (N,O-bis(trimethylsilyl)-trifluoroacetamide (available from Aldrich). The product is analysed by temperature and pressure programmed gas chromatography (GC) employing on-column injection and a SIMDIST fused silica capillary column. Flame ionization detection and an internal normalization method of quantification were employed.

The average number of moles of EO per molecule in the ethoxylate and the amount of residual (free) alcohols were typically 1.0 and 42.9 %wt., respectively. Comparative Example 15 - Preparation of a 3 % wt. DMC catalyst slurry in NEODOL 1 alcohol

29.3 g of technical grade zinc chloride (220 mmol zinc chloride, containing 2.2 mmol zinc oxide) and 8.8 mmol of zinc oxide were dissolved in 100 g of de-ionised water at room temperature. The resulting solution was charged to a 1 litre glass reactor, equipped with a triple pitched bladed turbine. Subsequently, 117 g of TBA and 95 g of de-ionised water were added.

A second solution, containing 12 g potassium hexacyanocobaltate dissolved in 225 g of de-ionised water, was added, over the course of 30 minutes, to the zinc chloride solution. The reactor contents were well stirred during the addition. After the addition the reactor contents were stirred for a subsequent 30 minutes and then allowed to stand overnight.

The following day, MTBE (12 %wt. on the basis of the reactor contents) was added and the reactor contents were stirred for 5 minutes. The reactor contents were then allowed to stand for 30 minutes, after which time, the lower (aqueous) liquid layer of the reactor contents (369 g) were removed.

A 25/75 wt./wt. mixture of TBA and de-ionised water (369 g) was introduced to the reactor. The reactor contents were stirred for 5 minutes, and then were allowed to stand for 30 minutes, after which time, the lower (aqueous) liquid layer of the reactor contents (307 g) was removed.

A 25/75 wt./wt. mixture of TBA and de-ionised water (307 g), with an additional MTBE (2.5 %wt. on initial reactor contents) was introduced to the reactor. The reactor contents were stirred for 5 minutes and then allowed to stand for 30 minutes, after which time, the lower (aqueous) liquid layer of the reactor contents (268 g) were removed.

TBA (268 g) was subsequently introduced and the reactor contents were stirred for 30 minutes.

The solids content of the resulting suspension was measured. The measurement was performed by weighing a sample of the suspension and subsequently removing the volatile components (MTBE, TBA and water) by stripping, under vacuum and a nitrogen purge at 50 °C, until a constant weight was obtained. Based on the solids content, NEODOL 1, a C₁₁ primary alcohol composition commercially available from The Shell Chemical Company, was added to obtain a 3 %wt. solution of zinc hexacyanocobaltate complex in NEODOL 1.

The reactor contents were stirred for 30 minutes, after which time, the MTBE, TBA and water were removed, by stripping at 60 °C and 50 mbar.

The resulting product had a water content of less than 0.5 %wt.

### Comparative Example 16 - Preparation of an ethoxylated C12/C13 alcohol having an average of 1 ethyleneoxy group per molecule by using a DMC catalyst

A 5-litre stirred tank reactor was charged with 3104 g of NEODOL 23, and 6.36 g of the catalyst slurry in NEODOL 1, formed in Comparative Example 15. Under constant stirring, the reactor tank was flushed three times with nitrogen, by raising the pressure within the reactor tank to 5 bara and subsequently releasing the pressure to atmospheric pressure. The reactor contents were heated, under a nitrogen atmosphere, to a temperature of 130 °C and subsequently stripped by applying a vacuum and a nitrogen purge at a pressure of 40 mbara for 15 minutes to remove the last traces of water. Then the pressure of nitrogen was increased to 2.4 bara.

704 g of ethylene oxide (EO) was introduced to the reactor contents such that during the addition of the first ca. 5 % of the EO, the pressure within the tank reactor did not exceed 3.8 bara. The induction time was 0 minutes, since the ethoxylation reaction started immediately after dosing the first EO. The remaining EO was subsequently added linearly over a 0.5-hour period, during which time the pressure increased due to the compression of the nitrogen gas-cap in the reactor.

After all of the EO had been introduced into the reactor, the reactor contents were held at the reaction temperature for 5 minutes, allowing the reaction of residual EO. Subsequently, any residual EO was stripped off with nitrogen at 40 mbara for 10 minutes. Then the reactor contents were allowed to cool to a temperature of 80 °C. The total amount of product composition formed, which was only very slightly hazy, was 3800 g.

Measurement of the average number of moles of EO per mole of NEODOL 23 and the level of residual (free) alcohol was performed using the gas chromatographic method described in Comparative Example 14. The average number of moles of EO per molecule in the thus-prepared NEODOL 23-1 and the amount of residual (free) alcohols were 1.03 and 37.5 %wt., respectively.

### Comparative Example 17 - Preparation of an ethoxylated secondary alcohol, ethoxylated 2-undecanol having an average of 1 ethyleneoxy group per molecule by using a DMC catalyst

The DMC-catalysed ethoxylation of 2-undecanol (available from Aldrich) was carried out in a 1-litre reactor according to the process described in detail in Comparative Example 16. In this case the induction time was about 20 minutes, and the time necessary to react the required amount of ethylene oxide was 70 minutes.

Measurement of the average number of moles of EO per mole of 2-undecanol and residual (free) secondary alcohol was performed using the gas chromatographic method described in Comparative Example 14. The average number of moles of EO per molecule in the thus-prepared ethoxylate of 2-undecanol (2-undecanol-lEO) and the amount of residual (free) secondary alcohol were 0.91 and 50.8 %wt., respectively.

### Comparative Example 18 - Preparation of an ethoxylate derived from the secondary alcohol 2-undecanol, having an average of about 6 EO groups per molecule, produced by acid catalysis

A "Teflon" bottle, equipped with a magnetic stirring bar and immersed in a (water) cooling bath, was charged with 2-undecanol (8.92 g), boric acid (55 mg) and 1.09 g of a solution of hydrogen fluoride (472 mg) in 2-undecanol (10.01 g). The bottle was then heated in an oil bath to 130°C. Ethylene oxide was added in the gas-phase at atmospheric pressure, at such a rate that the temperature did not exceed 130 °C (+/- 5 °C). After 1.5 hours, 17.6 g of ethylene oxide had been consumed. This corresponds with a degree of ethoxylation of 6.92 (on intake). EO dosing was then stopped and the mixture was stirred at 130°C for another 30 minutes and after cooling to ambient temperature the product was treated with diethanol amine (50 mg).

Measurement of the average number of moles of ethylene oxide per mole of 2-undecanol, the ethoxylate distribution and residual (free) alcohol was performed using high performance liquid chromatography (HPLC). The technique for these measurements involved derivatising the ethoxylated alcohol using 4-nitrobenzoylchloride. The product was then analysed by Gradient Elution High Performance Liquid Chromatography using a Polygosil Amino stationary phase with an isohexane/ ethylacetate/acetonitrile mobile phase. Detection was performed by ultra-violet absorbance. Quantification was by means of an internal normalisation technique.

The average number of moles of EO per molecule was 6.2, the level of free alcohol was 0.5 %wt. The level of 1,4-dioxane was determined by the GC method described in EP 1,663,928 B1 (Example 3, line 0108) and amounted to 16,000 ppm (wt./wt.).

The conversions of ethylsulfation of primary and secondary alcohols are generally higher than those obtained using a two-step process of ethoxylation using 1 equivalent of ethylene oxide, followed by sulfation. No distribution of alcohol ethoxylates and subsequently of alcohol ethoxysulphates, including alcohol sulphate itself, is formed. The absence of the latter constituent in alcohol ethoxysulfates is known to give rise to superior performance properties, such as calcium-tolerance, detergency and handleability.

## Claims

1. Use of an alkylene sulfate for reducing the level of at least one undesirable by-product in the formation of alkoxysulfates from one or more nucleophile.

2. Use according to claim 1, wherein the alkylene sulfate is ethylene sulfate, 1,2-propylene sulfate or 1,3-propylene sulfate.

3. Use according to claim 1 or claim 2, wherein the undesirable by-products are the corresponding nucleophile(s) and its sulphate(s), a distribution of heavily alkoxylated nucleophile(s) having more than 3 alkoxylate units per mole of nucleophile, and their sulfate(s) and/or 1,4-dioxane(s).

4. Use according to any one of claims 1 to 3, wherein the nucleophile is of the general formula (III)
R-XH (III),
wherein R refers to a group having a linear or branched hydrocarbyl backbone, or wherein the nucleophile is a tertiary amine of formula R⁰-N, wherein R⁰ refers to three groups that may be the same or different and are each, independently a group having a linear or branched hydrocarbyl backbone.

5. Use according to any one of claims 1 to 4, wherein the nucleophile of general formula (III) is an alcohol of general formula
R-OH,
wherein R refers to a group having a linear or branched hydrocarbyl backbone.

6. Use according to Claim 5, wherein the nucleophile is a primary or secondary alcohol having from 9 to 30 carbon atoms.

7. Use according to any one of claims 1 to 3, wherein the nucleophile is an alcohol of general formula (VII) 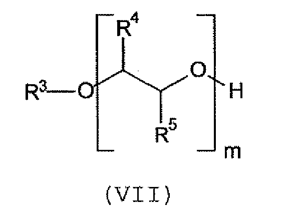 wherein, R³ refers to any suitable group, such that R³-OH is an alcohol and, wherein R⁴ and R⁵ are each, independently, hydrogen or an alkyl group.

8. Use according to Claim 7, wherein R³ is a group having a linear or branched hydrocarbyl backbone.
